# EUROPEAN PATENT APPLICATION

(11) **EP 4 591 916 A1**
(43) Date of publication of application: **30.07.2025**
(21) Application number: 24213461.7
(22) Date of filing: 16.11.2024
(51) Int. Cl.: A61M 25/10

(54) **MASKING MEMBER AND METHOD OF FORMING COATING LAYER**

(30) Priority: 24.01.2024 JP 2024008623
(71) Applicant: Terumo Kabushiki Kaisha, Tokyo, 1510072 (JP)
(72) Inventor: KOINUMA, Naoki, Shizuoka, 418-0015 (JP); ARISAKA, Akio, Shizuoka, 418-0015 (JP)
(74) Representative: KIPA AB

(57) **Abstract**

A masking member is to be used for masking a predetermined position of a balloon when a coating agent to be cured by electron beam irradiation is applied to the balloon, and the masking member includes a lumen continuously extending by a predetermined length along an extending direction of the balloon, and has a thickness of 50 µm or more.

## Description

### BACKGROUND

### Technical Field

The present invention relates to a masking member and a method of forming a coating layer.

### Related Art

For achieving smooth movement of a balloon catheter in the patient's body, a coating agent (for example, a coating liquid containing a hydrophilic polymer that forms a lubricating coating layer) may be applied to the surface of the balloon catheter to form a coating layer on the surface of the balloon catheter.

There are various methods of curing a coating agent applied to a balloon catheter, and a curing method may be used in which electron beam irradiation is used to shorten the time required for curing a coating agent.

A coating layer formed on a balloon catheter is required to exhibit coating performance excellent in durability in order to improve the arrival and passage of the balloon to the lesion. However, for example, in a product having a relatively short balloon length, a slip of the balloon at the time of expanding a lesion such as a stenosis makes it difficult to expand the lesion effectively. Therefore, when a coating agent is applied to a balloon portion of such a balloon catheter, a masking member may be disposed on the balloon to provide a predetermined position of the balloon with a non-coated region where a coating layer is not formed.

For example, WO 2017/18360 A discloses a masking member that is disposed so as to cover a predetermined site of a medical instrument such as a guide wire in order to form a surface lubricating layer (coating layer) of a predetermined pattern on the medical instrument.

### SUMMARY

One of considerations at the time of selecting a masking member to be used for forming a coating layer on a balloon is electron beam-permeability. For example, in the case of forming a coating layer using a masking member as described above, the masking member is fixed to a balloon to set a non-coated region where the coating agent is not applied. Then, in the state where the masking member is fixed to the balloon, the coating agent is applied to a coating target region of a balloon catheter including the balloon by dipping or the like. After applying the coating agent, the balloon catheter is irradiated with an electron beam for curing of the coating agent in the state where the masking member is fixed to the non-coated region of the balloon. At this time, if the electron beam is excessively transmitted through the masking member and reaches the non-coated region of the balloon, the electron beam causes an oxidation reaction or a molecular chain breakage, and thus the resin constituting the balloon may deteriorate to cause a product defect such as a change in balloon distention or deterioration of pressure resistance performance.

In order to solve a problem as described above, the inventors of the present invention have invented a masking member capable of suppressing an excessive electron beam influence due to electron beam irradiation on a masked non-coated region of a balloon, and a method of forming a coating layer using the masking member.

An object of the present invention is to provide a masking member capable of suppressing an excessive electron beam influence due to electron beam irradiation on a masked non-coated region of a balloon, and to provide a method of forming a coating layer using the masking member.

The present invention is achieved by any one of the following units (1) to (10).
(1) A masking member to be used for masking a predetermined position of a balloon when a coating agent to be cured by electron beam irradiation is applied to the balloon, the masking member including
   a lumen continuously extending by a predetermined length along an extending direction of the balloon,
   the masking member having a thickness of 50 µm or more.
(2) The masking member according to the unit (1), including a resin material having a density of 0.90 g/cm³ or more and 1.39 g/cm³ or less, or a metal material having a density of 7.75 g/cm³ or more and 21.45 g/cm³ or less.
(3) The masking member according to the unit (2), wherein the resin material includes an aromatic resin.
(4) The masking member according to the unit (2) or (3), wherein
   the resin material is polystyrene, polyether ether ketone, or polyethylene terephthalate, and
   the metal material is lead, stainless steel, iron, platinum, or gold.
(5) The masking member according to any one of the units (1) to (4), wherein the thickness is 350 µm or less.
(6) The masking member according to any one of the units (1) to (5), formed into a cylindrical shape having:
   an inner diameter of 0.7 mm or more and 6.0 mm or less;
   an outer diameter of 0.8 mm or more and 6.7 mm or less; and
   a length along the extending direction of the balloon of 5.0 mm or more and 50 mm or less.
(7) The masking member according to any one of the units (1) to (6), wherein the length along the extending direction of the balloon is equal to or less than a length of a straight portion of the balloon.
(8) A method of forming a coating layer, the method for formation of a coating layer at a predetermined position of a balloon catheter using the masking member according to any one of the units (1) to (7), the method including:
   fixing the masking member to at least a portion of a balloon of the balloon catheter to mask at least the portion of the balloon;
   covering the predetermined position of the balloon catheter with the coating agent; and
   irradiating the predetermined position of the balloon catheter covered with the coating agent with an electron beam in a state where the masking member is fixed to the balloon.
(9) The method of forming a coating layer according to the unit (8), wherein the masking member is fixed to the balloon in a state where the masking member covers at least a straight portion of the balloon.
(10) The method of forming a coating layer according to the unit (8) or (9), wherein
   the masking member is disposed over an outer periphery of the balloon in a state where the balloon is deflated,
   the masking member is fixed to the balloon by expanding and deforming the balloon to a diameter smaller than a maximum expansion diameter, and
   after irradiating with the electron beam, the balloon is deflated and detached from the masking member.

According to the masking member and the method of forming a coating layer according to the present invention, when a coating agent applied to a balloon catheter is cured, it is possible to inhibit an electron beam applied to the balloon catheter from reaching a non-coated region of the masked balloon. Therefore, it is possible to prevent an excessive electron beam influence due to electron beam irradiation on the non-coated region of the balloon, and it is possible to prevent deterioration of the product quality of the balloon catheter.

### BRIEF DESCRIPTION OF DRAWINGS

FIG. 1 is a view illustrating a balloon catheter according to an embodiment;
FIG. 2 is an axial direction sectional view of a vicinity of a distal portion of a balloon catheter according to an embodiment;
FIG. 3 is a sectional view for explanation of a method of forming a coating layer using a masking member according to an embodiment;
FIG. 4 is a sectional view for explanation of a method of forming a coating layer using a masking member according to an embodiment;
FIG. 5 is a view for explanation of a method of forming a coating layer using a masking member according to an embodiment;
FIG. 6 is a sectional view for explanation of a method of forming a coating layer using a masking member according to an embodiment; and
FIG. 7 is a sectional view for explanation of a method of forming a coating layer using a masking member according to an embodiment.

### DETAILED DESCRIPTION

Hereinafter, an embodiment of the present invention will be described with reference to the drawings. Dimensional ratios in the drawings are exaggerated for convenience of description, and may be different from actual ratios.

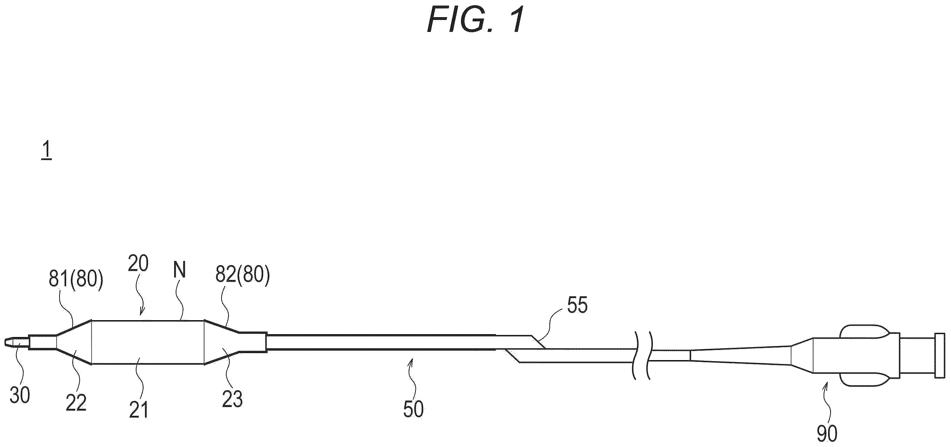

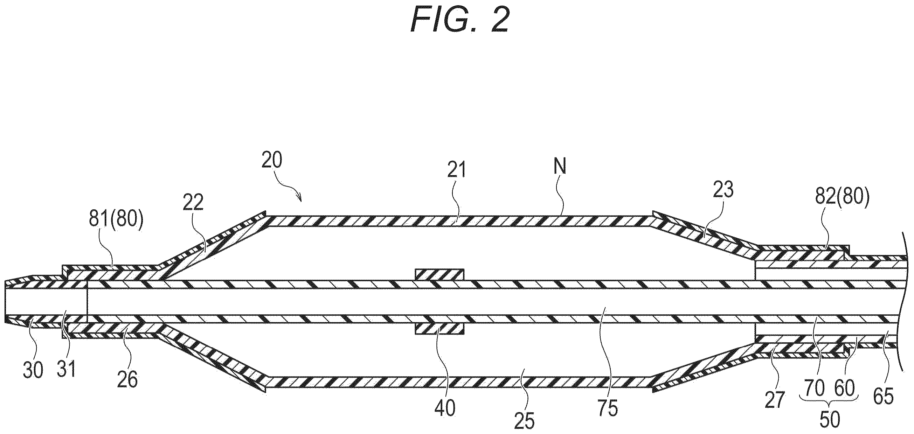

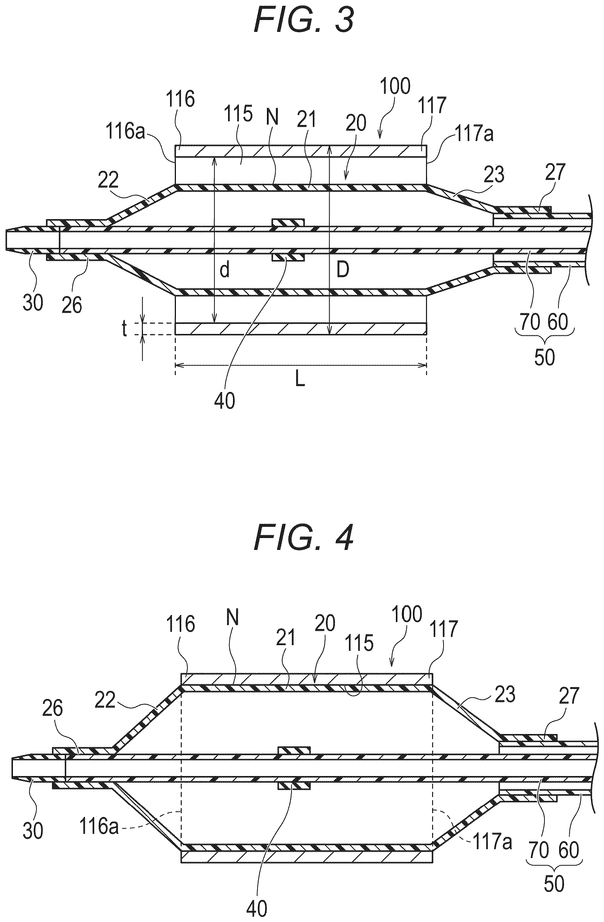

FIG. 1 is a view illustrating a whole configuration of a balloon catheter 1 according to an embodiment, FIG. 2 is a sectional view of a vicinity of a distal portion of the balloon catheter 1 according to an embodiment, and FIGS. 3 to 7 are views for explanation of a method of forming a coating layer 80 using a masking member 100 according to an embodiment.

### <Balloon Catheter 1>

FIGS. 1 and 2 illustrate a balloon catheter 1 according to the present embodiment. The balloon catheter 1 can be configured as, for example, a medical device used for percutaneous coronary intervention (PCI) or the like. However, the use of the balloon catheter 1 is not limited to the above, and the balloon catheter 1 can be configured as, for example, a medical device for the purpose of treating a lesion such as a stenosis formed in a biological organ such as another blood vessel, a bile duct, a trachea, an esophagus, another digestive tract, a urethra, an ear and nose lumen, or another organ.

As illustrated in FIG. 1, the balloon catheter 1 includes a balloon 20 that can be expanded and deflated as a fluid is injected and discharged, a distal end tip 30 disposed on a distal end side of the balloon 20, a flexible long shaft portion 50 to which the balloon 20 and the distal end tip 30 are fixed, a hub portion 90 disposed at a proximal portion of the shaft portion 50, and a coating layer 80 provided at a predetermined position of the balloon catheter 1.

In the present specification, a side to be introduced into a living body (a side on which the distal end tip 30 is disposed) in the balloon catheter 1 is referred to as a distal end side, and an end portion positioned on the distal end side in each member and component is referred to as a "distal portion". Furthermore, a side on which the hub portion 90 is disposed in the balloon catheter 1 is referred to as a proximal end side, and an end portion positioned on the proximal end side in each member and component is referred to as a "proximal portion". Furthermore, a direction from the distal portion to the proximal portion (or from the proximal portion to the distal portion) is referred to as an "axial direction (longitudinal direction)".

As illustrated in FIGS. 1 and 2, the balloon 20 includes a distal end fixing portion 26 and a proximal end fixing portion 27 fixed to the shaft portion 50, a straight portion 21 having a substantially linear sectional shape along the axial direction at the time of expansion, a distal end tapered portion 22 having a sectional shape in which the outer diameter gradually decreases from the distal end of the straight portion 21 toward the distal end fixing portion 26 side, and a proximal end tapered portion 23 having a sectional shape in which the outer diameter gradually decreases from the proximal end of the straight portion 21 toward the proximal end fixing portion 27 side.

The balloon 20 includes a film-like member having flexibility. The balloon 20 is fixed to the shaft portion 50 so as to define an internal space 25 into which a fluid can flow between the balloon 20 and an inner shaft 70 included in the shaft portion 50.

The straight portion 21 included in the balloon 20 is a portion that is disposed at a lesion such as a stenosis in a procedure using the balloon catheter 1 and applies pressure to the lesion as the balloon 20 expands.

Note that in the present specification, the balloon 20 is illustrated in an expanded state for convenience of briefly describing the present invention. However, the balloon 20 is actually folded into a predetermined shape in a state before expansion, and is prepared in a state of being wound around the shaft portion 50. As the shape of the balloon 20 such as a folded shape or a shape in a state of being wound around the shaft portion 50, a configuration known in the field of balloon catheters can be appropriately adopted.

The balloon 20 can be formed using, for example, a thermoplastic resin such as a polyolefin such as polyethylene, polypropylene, or an ethylene-propylene copolymer, a polyester such as polyethylene terephthalate, polyvinyl chloride, an ethylene-vinyl acetate copolymer, a crosslinked ethylene-vinyl acetate copolymer, or a polyurethane, a polyamide, a polyamide elastomer, a polystyrene elastomer, silicone rubber, or latex rubber.

To the hub portion 90 disposed at the proximal portion of the shaft portion 50, an indeflator can be connected for supplying various fluids (pressurizing media) to the internal space 25 of the balloon 20 and discharging the fluids from the internal space 25 of the balloon 20. The hub portion 90 can include a component known in the field of medical devices such as catheters.

The distal end tip 30 is fixed to the inner shaft 70 included in the shaft portion 50. The distal end tip 30 is disposed for the purpose of preventing damage to a biological organ (an inner wall of a blood vessel, or the like) when the distal end of the balloon catheter 1 comes into contact with the biological organ.

The distal end tip 30 can include a hollow member including a lumen 31 through which a guide wire can pass inside.

The distal end tip 30 can include, for example, a more flexible material than the shaft portion 50. Examples of a usable material to be included in the distal end tip 30 include polyolefins, polyvinyl chloride, polyamides, polyamide elastomers, and polyurethanes.

As illustrated in FIGS. 1 and 2, the shaft portion 50 includes an outer shaft 60 and the inner shaft 70.

The inner shaft 70 is disposed to pass through a lumen 65 of the outer shaft 60. A distal portion of the inner shaft 70 protrudes more toward the distal end side than the outer shaft 60 by a predetermined length. The distal end fixing portion 26 of the balloon 20 is fixed to the distal portion of the inner shaft 70, and the proximal end fixing portion 27 of the balloon 20 is fixed to a distal portion of the outer shaft 60.

The lumen 65 of the outer shaft 60 communicates with the internal space 25 of the balloon 20 to form a pressurizing medium lumen through which a fluid supplied via the hub portion 90 flows.

The balloon catheter 1 is configured as a rapid exchange catheter. As illustrated in FIG. 1, a proximal portion of the inner shaft 70 forms a guide wire port 55 serving as an inlet/outlet port through which a guide wire passes into the shaft portion 50. A lumen 75 of the inner shaft 70 forms a guide wire lumen through which a guide wire can pass. Note that the balloon catheter 1 can be configured as an over-the-wire catheter.

The outer shaft 60 and the inner shaft 70 can be formed using, for example, a polyolefin such as polyethylene, polypropylene, an ethylene-propylene copolymer, or an ethylene-vinyl acetate copolymer, a thermoplastic resin such as soft polyvinyl chloride, an elastomer such as a polyurethane elastomer, a polyamide elastomer, or a polyester elastomer, or a crystalline plastic such as a polyamide, crystalline polyethylene, or crystalline polypropylene.

As illustrated in FIG. 2, a contrast marker 40 having a contrast property can be disposed on the shaft portion 50 (inner shaft 70) at any position overlapping the balloon 20. The contrast marker 40 can include, for example, a known metal material having radiopacity.

The coating layer 80 is provided at a predetermined position of the balloon catheter 1. In the present embodiment, as illustrated in FIGS. 1 and 2, the coating layer 80 is provided from the distal portion of the balloon catheter 1 (the position where the distal end tip 30 is disposed) to the distal end of the straight portion 21 of the balloon 20 (the vicinity of the boundary between the straight portion 21 and the distal end tapered portion 22), and from the proximal end of the straight portion 21 of the balloon 20 (the vicinity of the boundary between the straight portion 21 and the proximal end tapered portion 23) to the vicinity of the distal end of the guide wire port 55. That is, as illustrated in FIG. 2, the straight portion 21 of the balloon 20 is a "non-coated region N" where the coating layer 80 cured by electron beam irradiation is not disposed.

Hereinafter, in the present embodiment, the coating layer 80 formed from the distal portion of the balloon catheter 1 to the distal end of the straight portion 21 of the balloon 20 is referred to as a "first coating layer 81". The coating layer 80 formed from the proximal end of the straight portion 21 of the balloon 20 to the vicinity of the distal end of the guide wire port 55 is referred to as a "second coating layer 82". In the present embodiment, the above two regions exemplify the region where the coating layer 80 is provided, but the position where the coating layer 80 is provided can be set to any position according to, for example, the product specification of the balloon catheter 1.

The "non-coated region N" means a region to which a coating agent 210 described below is not applied (region where the coating layer 80 is not formed), and does not define that another coating layer such as a drug layer is not provided.

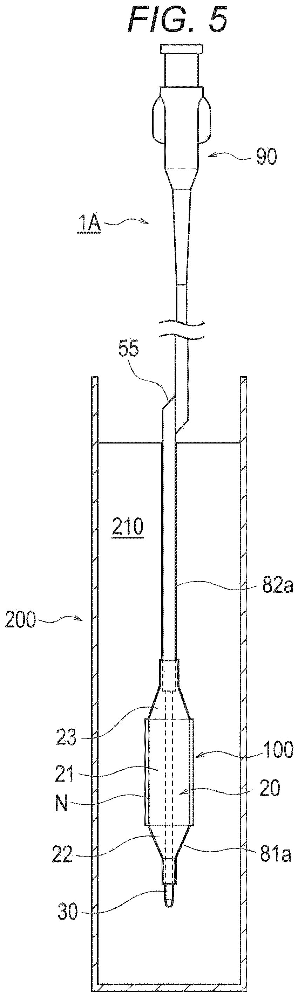

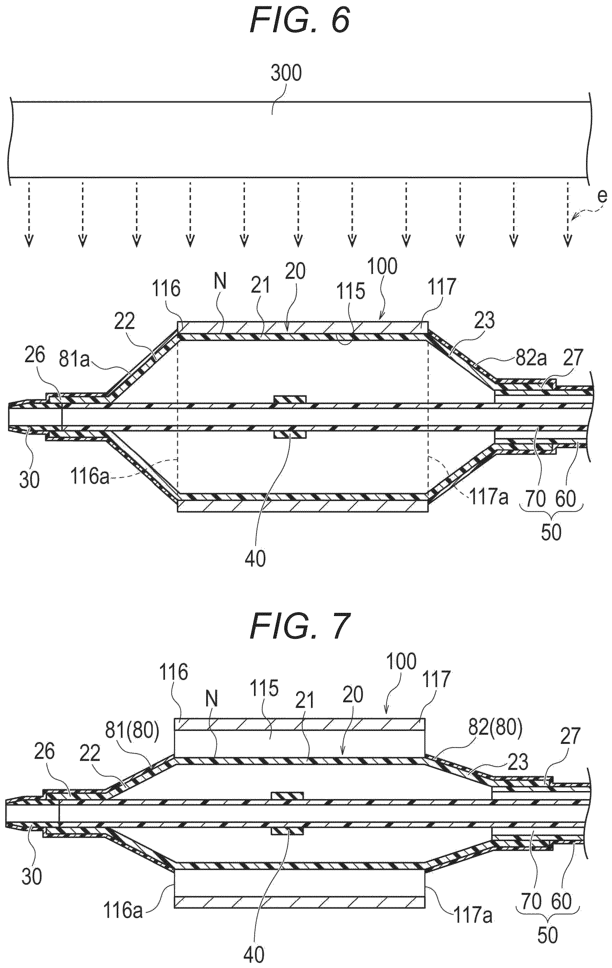

The coating layer 80 is formed by irradiating the coating agent 210 (see FIG. 5) applied to the balloon catheter 1 with an electron beam to cure the coating agent 210 (see FIGS. 5 and 6). As described below, in a method of forming the coating layer 80 for formation of the straight portion 21 as the non-coated region N, the masking member 100 (see FIGS. 3, 5, and the like) is used.

The coating agent 210 that constitutes the coating layer 80 (the first coating layer 81 and the second coating layer 82) may be any agent as long as it absorbs water and exhibits lubricity. Examples of the coating agent 210 include hydrophilic materials. Specific examples will be described below. In the specific examples, the term "(meth)acryl" refers to both acryl and methacryl. Therefore, for example, the term "(meth)acrylic acid" refers to both acrylic acid and methacrylic acid. Similarly, the term "(meth)acryloyl" refers to both acryloyl and methacryloyl. Therefore, for example, the term "(meth)acryloyl group" refers to both an acryloyl group and a methacryloyl group.

Examples of the hydrophilic material constituting the coating agent 210 include hydrophilic polymers such as polyvinyl pyrrolidone, polyvinyl alcohol, polyethylene oxide-based polymers, cellulose-based polymers such as carboxymethyl cellulose, acrylamide-based polymers such as polyacrylamide and polydimethylacrylamide, hyaluronic acid, polyacrylic acid, maleic anhydride-based polymers such as a maleic anhydride-methyl vinyl ether copolymer, watersoluble nylon (registered trademark), and derivatives thereof.

For firmly fixing the hydrophilic polymer to a predetermined site of the balloon catheter 1 (for example, a site other than the straight portion 21 of the balloon 20 or the shaft portion 50), the hydrophilic material constituting the coating agent 210 may be a hydrophilic copolymer including a monomer having a reactive functional group (hereinafter, also referred to as a "reactive monomer") and a hydrophilic monomer. The term "reactive functional group" as used herein refers to a functional group capable of a crosslinking reaction with another monomer or capable of a reaction (bonding) with the surface of the balloon catheter 1 by electron beam irradiation or the like.

The reactive functional group is not particularly limited, and may be a functional group such as an epoxy group, an acid halide group, an aldehyde group, an isocyanate group, an acid anhydride group, a vinyl group, or a (meth)acryloyl group. One or a plurality of these reactive functional groups may be present in the reactive monomer.

It is preferable that the reactive monomer used in the present invention have a reactive functional group and be more hydrophobic in a body fluid or an aqueous solvent than at least the hydrophilic monomer used in the production of the copolymer. Specific examples of such a reactive monomer include monomers having an epoxy group in the molecule, such as glycidyl acrylate, glycidyl methacrylate (GMA), methyl glycidyl methacrylate, and allyl glycidyl ether; monomers having an acid halide group in the molecule, such as (meth)acrylic acid chloride, (meth)acrylic acid bromide, and (meth)acrylic acid iodide; monomers having an aldehyde group in the molecule, such as (meth)acrylaldehyde, crotonaldehyde, acrolein, and methacrolein; monomers having an isocyanate group in the molecule, such as (meth)acryloyloxymethyl isocyanate, (meth)acryloyloxyethyl isocyanate, (meth)acryloyloxypropyl isocyanate, and (meth)acryloyl isocyanate; monomers having an acid anhydride group in the molecule, such as maleic anhydride, itaconic anhydride, and citraconic anhydride; and monomers having a vinyl group in the molecule, such as vinyl chloride. The reactive monomer is preferably a monomer having an epoxy group in the molecule, such as glycidyl acrylate, glycidyl methacrylate (GMA), methyl glycidyl methacrylate, or allyl glycidyl ether. These reactive monomers can be used singly or in combination of two or more kinds thereof.

The hydrophilic monomer is not particularly limited, and examples of the hydrophilic monomer include acrylic acid, methacrylic acid, N-methylacrylamide, N,N-dimethylacrylamide (DMAA), acrylamide, acryloylmorpholine, N,N-dimethylaminoethyl acrylate, vinylpyrrolidone, 2-methacryloyloxyethyl phosphorylcholine, 2-methacryloyloxyethyl-D-glycoside, 2-methacryloyloxyethyl-D-mannoside, vinyl methyl ether, 2-hydroxyethyl (meth)acrylate, 4-hydroxybutyl (meth)acrylate, 2-hydroxypropyl (meth)acrylate, 2-hydroxybutyl (meth)acrylate, 6-hydroxyhexyl (meth)acrylate, 1,4-cyclohexanedimethanol mono(meth)acrylate, 1-chloro-2-hydroxypropyl (meth)acrylate, diethylene glycol mono(meth)acrylate, 1,6-hexanediol mono(meth)acrylate, pentaerythritol tri(meth)acrylate, dipentaerythritol penta(meth)acrylate, neopentyl glycol mono(meth)acrylate, trimethylolpropane di(meth)acrylate, trimethylolethane di(meth)acrylate, 2-hydroxy-3-phenyloxypropyl (meth)acrylate, 4-hydroxycyclohexyl (meth)acrylate, 2-hydroxy-3-phenyloxy (meth)acrylate, 4-hydroxycyclohexyl (meth)acrylate, cyclohexanedimethanol mono(meth)acrylate, poly(ethylene glycol) methyl ether acrylate, and poly(ethylene glycol) methyl ether methacrylate. The hydrophilic monomer is preferably N,N-dimethylacrylamide, acrylamide, acrylic acid, methacrylic acid, N,N-dimethylaminoethyl acrylate, 2-hydroxyethyl acrylate, or vinylpyrrolidone. These hydrophilic monomers can be used singly or in combination of two or more kinds thereof.

For easily and firmly fixing the hydrophilic polymer to the balloon catheter 1, the hydrophilic material constituting the coating agent 210 may be a material including only a hydrophilic monomer. In this case, the hydrophilic monomer includes a (meth)acryloyl group capable of a crosslinking reaction with another hydrophilic monomer or capable of a reaction (bonding) with the surface of each portion of the balloon catheter 1 by electron beam irradiation. Such a hydrophilic monomer is, for example, a hydrophilic monomer including a (meth)acryloyl group among the above-described hydrophilic monomers. The material including only a hydrophilic monomer is preferably a material including a single hydrophilic monomer including a (meth)acryloyl group or including a combination of two or more kinds among the above-described hydrophilic monomers.

The viscosity of the coating agent 210 can be set to any value according to the material to be used, the environment when the method of forming the coating layer 80 is carried out, and the like, and can be set to, for example, a value of 15 mPa·s or more and 25 Pa·s or less.

### <Masking Member 100>

As illustrated in FIGS. 3 to 7, the masking member 100 is used to mask a predetermined position of the balloon 20 at the time of applying the coating agent 210 to be cured by electron beam irradiation.

In the present embodiment, a usage example will be described in which the masking member 100 is used in a method of forming the coating layer 80 for formation of the non-coated region N where the coating layer 80 is not formed on the straight portion 21 of the balloon 20 (see FIGS. 3 to 7). That is, the masking member 100 is fixed to the balloon 20 so as to cover the whole or at least a portion of the straight portion 21 in a method of forming the coating layer 80 described below.

As illustrated in FIG. 3, the masking member 100 includes a lumen 115 continuously extending by a predetermined length along the extending direction of the balloon 20 (the same direction as the axial direction of the shaft portion 50).

The masking member 100 includes a hollow member having an inner diameter d and an outer diameter D that are substantially constant in the axial direction. The masking member 100 has a distal portion 116, in the axial direction, that is provided with a distal end opening 116a communicating with the lumen 115. The masking member 100 has a proximal portion 117, in the axial direction, that is provided with a proximal end opening 117a communicating with the lumen 115.

The masking member 100 includes the lumen 115 continuously extending by a predetermined length along the axial direction as described above, and therefore in a state of being fixed to the balloon 20, the masking member 100 can continuously cover an any range along the axial direction of the balloon 20 along the axial direction. Thus, using the masking member 100 enables formation of the non-coated region N continuously extending along an any range along the axial direction of the balloon 20.

The masking member 100 can have a thickness t of 50 µm or more. In a case where the masking member 100 has a thickness t of 50 µm or more, if the outer surface side of the masking member 100 is irradiated with an electron beam having a predetermined energy in a state where the masking member 100 is fixed to the straight portion 21 of the balloon 20 (see FIG. 6), it is possible to prevent a product defect, such as a change in balloon distention or deterioration of pressure resistance performance, caused by an oxidation reaction or a molecular chain breakage due to the electron beam and the resulting deterioration of the resin constituting the balloon.

The thickness t of the masking member 100 is preferably 50 µm or more in consideration of the electron beam influence on the balloon 20 as described above. Meanwhile, if the thickness t is excessively large, when the coating agent 210 is applied to the balloon catheter 1 as described below, the thickness t of the masking member 100 has an influence on generation of a step due to a liquid pool of the coating agent 210 in the vicinity of the boundary between the non-coated region N and the coated region to which the coating agent 210 is applied. If such a step is generated, the coating layer 80 may be peeled off and fine particles of the material constituting the coating layer 80 may be scattered at the time of using the balloon catheter 1. The thickness of the masking member 100 is preferably 80 µm or less in order to prevent formation of a step due to a liquid pool as described above.

From the viewpoint of achieving both reduction of the electron beam influence on the balloon 20 and prevention of a liquid pool, the masking member 100 is, for example, preferably larger than 50 µm and smaller than 350 µm, and more preferably 50 µm or more and 150 µm or less.

The masking member 100 can include, for example, a resin material having a density of 0.90 g/cm³ or more and 1.39 g/cm³ or less.

If the masking member 100 includes a resin material having a density as described above, the masking member 100 can effectively suppress an excessive influence of an electron beam transmitted through the masking member 100 on the non-coated region N.

The resin material is preferably a material having high resistance to a radiation such as an electron beam, specifically, a material that is less likely to generate radicals and has high stability (low reactivity) or a material that is less likely to crosslink and decompose. More specifically, the resin material preferably has a structure including a benzene ring, and as an example, an aromatic resin can be suitably used.

As the aromatic resin, for example, any one can be suitably selected from polystyrene having a density of 1.04 g/cm³ or more and 1.07 g/cm³ or less, polyethylene terephthalate having a density of 1.38 g/cm³ or more and 1.39 g/cm³ or less, and polyether ether ketone having a density of 1.3 g/cm³.

Alternatively, the masking member 100 can include, for example, a metal material having a density of 7.75 g/cm³ or more and 21.45 g/cm³ or less.

If the masking member 100 includes a metal material having a density as described above, the masking member 100 can effectively suppress an excessive influence of an electron beam transmitted through the masking member 100 on the non-coated region N, similarly to the masking member 100 including a resin material having a predetermined density described above.

As the metal material, for example, any one can be suitably selected from platinum having a density of 21.45 g/cm³, lead having a density of 11.35 g/cm³, gold having a density of 19.32 g/cm³, and stainless steel (SUS303, SUS304, SUS316, SUS430, SUS444, SUS403, SUS410, SUS440) having a density of 7.75 or more and 7.93 g/cm³ or less.

In a case where the masking member 100 includes a metal material, the distal portion 116 and the proximal portion 117 of the masking member 100 are preferably subjected to chamfering to prevent the balloon 20 from being damaged when the masking member 100 is disposed on and fixed to the balloon 20.

The masking member 100 can be formed into a cylindrical shape having, for example, an inner diameter d of 0.7 mm or more and 6.0 mm or less, an outer diameter D of 0.8 mm or more and 6.7 mm or less, and a length along the extending direction of the balloon 20 (length in the axial direction) L of 5.0 mm or more and 50 mm or less.

In the masking member 100, the non-coated region N can be formed in any range of the balloon 20 of the balloon catheter 1 if the dimensions of each portion of the masking member 100 are set as described above.

In a case where the non-coated region N is formed in the straight portion 21 of the balloon 20, the length L of the masking member 100 can be equal to or less than the length of the straight portion 21 of the balloon 20. The length of the straight portion 21 of the balloon 20 can be set to various lengths according to the kind of the product of the balloon catheter 1, and in the present embodiment, the length is, for example, 5 mm or more and 50 mm or less. Therefore, in a case where the non-coated region N is formed in the whole of the straight portion 21 in the axial direction or a portion of the straight portion 21 in the axial direction in the balloon 20, the length L of the masking member 100 is preferably, for example, 50 mm or less.

### <Method of Forming Coating Layer>

Next, a method of forming the coating layer 80 according to the present embodiment will be described.

In the method of forming the coating layer 80 according to the present embodiment, the coating layer 80 is formed at a predetermined position of the balloon catheter 1 using the masking member 100. In the present embodiment, as described above, a method of forming the coating layer 80 will be described in which the first coating layer 81 and the second coating layer 82 (See FIGS. 1 and 2) are formed on the balloon catheter 1 and the non-coated region N is formed over the whole (whole length) of the straight portion 21 in the axial direction in the balloon 20.

As illustrated in FIG. 3, the masking member 100 is disposed over an outer periphery of the balloon 20 in a state where the balloon 20 is deflated. At this time, the straight portion 21 of the balloon 20 is inserted into the lumen 115 of the masking member 100 through the proximal end opening 117a of the masking member 100, and the straight portion 21 is covered with the masking member 100.

Next, as illustrated in FIG. 4, the balloon 20 is expanded and deformed to a diameter smaller than the maximum expansion diameter (rated burst pressure: RBP), and thus the masking member 100 is fixed to the balloon 20. A predetermined injection device is connected to the above-described hub portion 90, and thus the balloon 20 can be expanded. The balloon 20 disposed in the lumen 115 of the masking member 100 is expanded, and thus the masking member 100 can be fixed to the straight portion 21 using the expansion force of the balloon 20. By fixing the masking member 100 to the straight portion 21, the straight portion 21 can be masked. The masking member 100 is fixed to the balloon 20 by expanding the balloon 20 as described above, and therefore preparation and use of another device such as a jig is unnecessary for fixing the masking member 100 to the balloon 20. Therefore, the work efficiency can be improved, and the manufacturing cost can be reduced. The maximum expansion diameter of the balloon 20 can be set to any size based on the kind and the like of the balloon catheter 1, and is, for example, 0.75 mm or more and 6.00 mm or less in the present embodiment.

Next, the coating agent 210 is applied to any range on the distal end side other than the non-coated region N (straight portion 21) of the balloon catheter 1. The method of applying the coating agent 210 is not particularly limited, and for example, dip coating can be adopted. As illustrated in FIG. 5, a preparation article 1A of the balloon catheter 1 (intermediate product before completion of the coating layer 80) is immersed from the distal end side in the coating agent 210 stored in a storage tank 200, and thus the coating agent 210 is applied to a first coating region 81a corresponding to the first coating layer 81 and a second coating region 82a corresponding to the second coating layer 82. Since the masking member 100 is fixed to the straight portion 21 of the balloon 20 of the preparation article 1A, the coating agent 210 can be prevented from being applied to the straight portion 21 of the balloon 20 at the time of immersing the preparation article 1A in the coating agent 210.

After applying the coating agent 210 to a predetermined region of the preparation article 1A, the preparation article 1A is taken out from the storage tank 200.

Next, as illustrated in FIG. 6, in order to cure the coating agent 210 applied to the preparation article 1A, the preparation article 1A is irradiated with an electron beam from an electron beam irradiation unit 300 included in a known electron beam irradiation device (for electron beam irradiation indicated by the arrow e in the drawing). At the time of irradiating the preparation article 1A with an electron beam, the masking member 100 is kept to be fixed to the straight portion 21 of the balloon 20. When the preparation article 1A is irradiated with an electron beam, the coating agent 210 irradiated with the electron beam is cured. As a result of curing of the coating agent 210, the first coating layer 81 and the second coating layer 82 can be formed at predetermined positions of the balloon catheter 1. At the time of irradiating the balloon catheter 1 with an electron beam, the masking member 100 is fixed to the straight portion 21 of the balloon 20, and therefore an excessive electron beam influence on the straight portion 21 of the balloon 20 can be effectively suppressed.

The masking member 100 can inhibit an electron beam from reaching the straight portion 21 of the balloon 20, and thus the masking can prevent the coating agent 210 from being applied to the straight portion 21 in the step of applying the coating agent 210 to the preparation article 1A (dipping step), and can prevent an excessive electron beam influence due to electron beam irradiation on the straight portion 21 in the next step of irradiating with an electron beam. Therefore, in the method of forming the coating layer 80 using the masking member 100, the masking member 100 is kept to be fixed to the straight portion 21 after completion of the step of applying the coating agent 210 to the preparation article 1A, and thus the step of irradiating with an electron beam can be started without, for example, an operation of replacing the masking member 100 with another protective member for protection of the straight portion 21 from electron beam irradiation. Therefore, the number of jigs used in the method of forming the coating layer 80 can be reduced, and the manufacturing process can be simplified.

Various conditions at the time of curing the coating agent 210 by electron beam irradiation are not particularly limited as long as the coating layer 80 formed by curing the coating agent 210 including the above-described material can be formed at a predetermined position (in the present embodiment, positions corresponding to the regions where the first coating layer 81 and the second coating layer 82 are formed, respectively) of the balloon catheter 1. For example, the energy of the electron beam emitted from the electron beam irradiation unit 300 can be set to 1 keV or more and 100 keV or less, and can be preferably set to 50 keV or more and 70 keV or less. The irradiation distance (distance from the emission end of the electron beam irradiation unit 300 to the surface of the coating agent 210 positioned at the irradiation target position) can be 1 mm or more and 50 mm or less, and can be preferably 10 mm.

Next, as illustrated in FIG. 7, after irradiation with an electron beam, the balloon 20 is deflated and removed from the masking member 100.

According to the above operation procedure, any position (for example, the whole or a portion of the straight portion 21 in the axial direction) of the balloon 20 of the balloon catheter 1 is masked with the masking member 100, and thus the non-coated region N where the coating layer 80 is not formed can be provided at any position described above. By using the masking member 100, it is possible to effectively suppress an electron beam influence due to electron beam irradiation on the non-coated region N, so that it is possible to suppress deterioration of the product quality of the balloon catheter 1. When the coating agent 210 is cured under the above-described conditions to form the coating layer 80, the masking member 100 can particularly effectively suppress an electron beam influence due to electron beam irradiation on the non-coated region N.

The above-described method of forming the coating layer 80 exemplifies a method of providing the non-coated region N over the whole of the straight portion 21 in the axial direction (extending direction) and the masking member 100 used in such a method, but the range where the non-coated region N is provided in the balloon 20 is only required to be at least a portion of the balloon 20 in the axial direction, and is not limited to only the whole of the straight portion 21 as described above.

The masking member 100 according to the present embodiment is the masking member 100 to be used for masking a predetermined position of the balloon 20 when the coating agent 210 to be cured by electron beam irradiation is applied to the balloon 20, and the masking member 100 includes the lumen 115 continuously extending by a predetermined length along the extending direction of the balloon 20 and has a thickness of 50 µm or more.

The method of forming the coating layer 80 according to the present embodiment is the method of forming the coating layer 80 for formation of the coating layer 80 at a predetermined position of the balloon catheter 1 using the masking member 100, and the method includes fixing the masking member 100 to at least a portion of the balloon 20 of the balloon catheter 1 to mask at least the portion of the balloon 20, covering the predetermined position of the balloon catheter 1 with the coating agent 210, and irradiating the predetermined position of the balloon catheter 1 covered with the coating agent 210 with an electron beam in a state where the masking member 100 is fixed to the balloon 20.

According to the masking member 100 and the method of forming the coating layer 80 according to the present embodiment, when the coating agent 210 applied to the balloon catheter 1 is cured, an electron beam applied to the balloon catheter 1 can be inhibited from reaching the non-coated region N of the masked balloon 20. Therefore, it is possible to prevent a change in distention or deterioration of pressure resistance performance in the balloon 20, caused by an oxidation reaction or a molecular chain breakage due to an electron beam and the resulting deterioration of the resin constituting the balloon 20. Furthermore, the non-coated region N can be provided at at least a portion (for example, the whole or a portion of the straight portion 21) of the balloon 20, and therefore it is possible to prevent excessive slip of the balloon 20 at the time of expanding a lesion using the balloon 20.

The masking member and the method of forming a coating layer according to the present invention is described above through the embodiment, but the present invention is not limited to only the configurations described in the embodiment, and can be appropriately changed on the basis of the description of the claims.

The balloon catheter can also be configured as, for example, a catheter for stent delivery in which a stent is mounted on a straight portion of a balloon. In a case where the balloon catheter is configured as a catheter for stent delivery, the stent can be crimped and held on the straight portion where a non-coated region is formed in the balloon. In the catheter for stent delivery configured as described above, the stent can be prevented from falling off from the balloon while the passage to a lesion or the like is maintained by the coating layer provided at a predetermined position of the balloon catheter, and the stent can be further appropriately placed at the lesion. The stent can also be configured as a drug eluting stent (DES) having a drug layer. In a case where the stent is configured as a DES, the effect of the drug on a lesion can be effectively exhibited.

The balloon catheter can also be configured using a drug coated balloon (DCB) in which a drug layer is disposed in a non-coated region of the balloon. In the DCB configured as described above, the drug layer disposed on the balloon can be prevented from peeling off while the passage to a lesion or the like is maintained by the coating layer provided at a predetermined site of the balloon catheter, and the balloon can be further appropriately delivered to the lesion. Therefore, the effect of the drug layer can be effectively exhibited.

## Claims

1. A masking member to be used for masking a predetermined position of a balloon when a coating agent to be cured by electron beam irradiation is applied to the balloon, the masking member comprising
a lumen continuously extending by a predetermined length along an extending direction of the balloon,
the masking member having a thickness of 50 µm or more.

2. The masking member according to claim 1, comprising a resin material having a density of 0.90 g/cm³ or more and 1.39 g/cm³ or less, or a metal material having a density of 7.75 g/cm³ or more and 21.45 g/cm³ or less.

3. The masking member according to claim 2, wherein the resin material includes an aromatic resin.

4. The masking member according to claim 2 or 3, wherein
the resin material is polystyrene, polyether ether ketone, or polyethylene terephthalate, and
the metal material is lead, stainless steel, iron, platinum, or gold.

5. The masking member according to claim 1, wherein the thickness is 350 µm or less.

6. The masking member according to claim 1, formed into a cylindrical shape having:
an inner diameter of 0.7 mm or more and 6.0 mm or less;
an outer diameter of 0.8 mm or more and 6.7 mm or less; and
a length along the extending direction of the balloon of 5.0 mm or more and 50 mm or less.

7. The masking member according to claim 6, wherein the length along the extending direction of the balloon is equal to or less than a length of a straight portion of the balloon.

8. A method of forming a coating layer, the method for formation of a coating layer at a predetermined position of a balloon catheter using the masking member according to claim 1, the method comprising:
fixing the masking member to at least a portion of a balloon of the balloon catheter to mask at least the portion of the balloon;
covering the predetermined position of the balloon catheter with the coating agent; and
irradiating the predetermined position of the balloon catheter covered with the coating agent with an electron beam in a state where the masking member is fixed to the balloon.

9. The method of forming a coating layer according to claim 8, wherein the masking member is fixed to the balloon in a state where the masking member covers at least a straight portion of the balloon.

10. The method of forming a coating layer according to claim 8 or 9, wherein
the masking member is disposed over an outer periphery of the balloon in a state where the balloon is deflated,
the masking member is fixed to the balloon by expanding and deforming the balloon to a diameter smaller than a maximum expansion diameter, and
after irradiating with the electron beam, the balloon is deflated and detached from the masking member.
